# EUROPEAN PATENT APPLICATION

(11) **EP 4 548 872 A1**
(43) Date of publication of application: **07.05.2025**
(21) Application number: 23845261.9
(22) Date of filing: 04.07.2023
(51) Int. Cl.: A61B 34/30

(54) **END EFFECTOR MECHANISM OF SURGICAL INSTRUMENT, AND SURGICAL INSTRUMENT**

(30) Priority: 26.07.2022 CN 202210883433
(71) Applicant: Cornerstone Technology (Shenzhen) Limited, Shenzhen Guandong 518066 (CN)
(72) Inventor: WANG, Zerui, Shenzhen, Guangdong 518066 (CN)
(74) Representative: Balder IP Law, S.L.
(86) International application number: PCT/CN2023/105723
(87) International publication number: WO 2024/022048

(57) **Abstract**

The present disclosure provides an end effector of a surgical instrument and the surgical instrument. The end effector includes a lower support member (180), at least one set of guide wheels, a middle support member (120), and an end effector tool (110). The middle support member includes a first support portion (121) for defining an effector axis (190) and a second support portion (122) for defining a guide wheel axis. The second support portion (122) is rotatable about the guide wheel axis with a set of guide wheels. The end effector tool (110) is connected to the lower support member (180) through the middle support member (120). The end effector tool (110) and the first support portion (121) are rotatable about the effector axis (190), and the first support portion (121) is located in a distal direction of the second support portion (122). The effector axis (190) and the guide wheel axis defined by the second support portion (122) are skew lines, and an included angle between the effector axis (190) and the guide wheel axis defined by the second support portion (122) is an acute angle.

## Description

### CROSS REFERENCE TO RELATED APPLICATIONS

This application claims priority to Chinese Patent Application No. CN202210883433.0, entitled "END EFFECTOR AND SURGICAL INSTRUMENT HAVING THE SAME," filed on July 26, 2022, which is hereby incorporated by reference in its entirety.

### TECHNICAL FIELD

The various embodiments described in this document relate in general to the technical field of medical devices, and more specifically to an end effector and a surgical instrument having the same.

### BACKGROUND

Wrist at a distal end of existing surgical instruments generally has three degrees of freedom, including pitch, yaw, and an action of an end effector tool. The action of the end effector tool includes, for example, gripping, grasping, or cutting, etc. In order to reduce the surgical wound while facilitating flexible operation within a narrow space at a target surgical site, such surgical instrument may generally have a relatively small diameter and adopt a wire-driven manner to transfer the motion from a proximal end of the surgical instrument to the wrist at the distal end.

### SUMMARY

A series of simplified forms of concepts are introduced in this section of the disclosure, which will be described in further detail in the following section of the disclosure. The section of the present disclosure is not intended to define critical features and essential features of the claimed technical solution, and also not intended to determine the scope of protection of the claimed technical solution.

To at least partially solve the above problems, an object of some embodiments of the present disclosure is to provide an end effector of a surgical instrument, including: a lower support member; at least one set of guide wheels disposed on the lower support member, where each set of guide wheels is rotatable about a corresponding guide wheel axis of at least one guide wheel axis relative to the lower support member; a middle support member, including a first support portion for defining an effector axis and a second support portion for defining one guide wheel axis of the at least one guide wheel axis, where the second support portion and one set of guide wheels of the at least one set of guide wheels are rotatable about the one guide wheel axis, where the first support portion is disposed in a distal direction of the second support portion; and an end effector tool, where the end effector tool is connected to the lower support member through the middle support member, and the end effector tool and the first support portion are rotatable about the effector axis. The effector axis and the one guide wheel axis defined by the second support portion are skew lines, and an included angle between the effector axis and the one guide wheel axis is an acute angle.

According to the end effector of the surgical instrument of the present disclosure, both of the effector axis corresponding to a yaw axis and the guide wheel axis corresponding to a pitch axis are skew lines and non-perpendicular, so that the driving wires extending downward from the end effector tool to the guide wheel sets are tangent to guiding structures of respective guide wheels with which respective driving wires cooperate. Therefore, when the end effector tool is operated, the pulling force of the driving wires may cause a radial force on the respective guide wheels, and the axial force exerted on the guide wheel is reduced. In an ideal situation, no axial force will be generated, which makes the overall force on the guide wheel relatively small. Moreover, since the axial force is reduced or not existed, the axial friction is also reduced or not existed. As a result, the total friction generated between the driving wires and the guide wheels is relatively small, thereby reducing the wear of the driving wires and improving the performance (such as precision) of the surgical instrument.

### BRIEF DESCRIPTION OF THE DRAWINGS

One or more embodiments are illustrated by pictures in the accompanying drawings corresponding thereto, and these illustrative illustrations are not intended to limit the embodiments. Elements bearing the same reference numerals in the accompanying drawings are designated as similar elements, and the figures in the accompanying drawings are not intended to limit the scale unless otherwise stated.
FIG. 1 is a perspective view of an end effector according to an embodiment of the present disclosure.
FIG. 2 is an exploded perspective view of the end effector shown in FIG. 1.
FIG. 3 is a side view of a first end effector portion shown in FIG. 1.
FIG. 4 is a perspective view of a first end effector portion shown in FIG. 1.
FIG. 5 is a perspective view of a middle support member shown in FIG. 1.
FIG. 6 is a top view of a middle support member shown in FIG. 1.
FIG. 7 is a top view of an upper guide wheel set shown in FIG. 1.
FIG. 8 is a perspective view of a lower support member shown in FIG. 1.
FIG. 9 is a perspective view of a first end effector portion with a driving wire shown in FIG. 1.
FIG. 10 is a side view of a first end effector portion with a driving wire shown in FIG. 1.
FIG. 11 is a perspective view of a first end effector portion, an upper guide wheel set, and a lower guide wheel set with a driving wire shown in FIG. 1.
FIG. 12 is a perspective view of a second end effector portion, an upper guide wheel set, and a lower guide wheel set with a driving wire shown in FIG. 1.
FIG. 13 is a perspective view of a first end effector portion, a second end effector portion, an upper guide wheel set, and a lower guide wheel set with driving wires shown in FIG. 1.
FIG. 14 is a perspective view of an end effector with a middle support member of a fork-shaped structure according to another embodiment of the present disclosure.
FIG. 15 is a side view of the end effector shown in FIG. 14.
FIG. 16 is a perspective view of a middle support member shown in FIG. 14.
FIG. 17 is a schematic structural diagram of a conventional surgical robot system.

### DETAILED DESCRIPTION OF THE EMBODIMENTS

In the following description, numerous specific details are given in order to provide a more thorough understanding of the present disclosure. However, it will be apparent to those skilled in the art that the present disclosure may be practiced without one or more of these details. In other examples, in order to avoid confusion with the present disclosure, some technical features well known in the art have not been described.

For a thorough understanding of the present disclosure, a detailed illustration will be set forth in the following description. It is obvious that the implementation of the embodiments of the present disclosure is not limited to specific details familiar to those skilled in the art. Preferred embodiments of the present disclosure are described in detail below, however, the present disclosure may have other embodiments in addition to these detailed descriptions.

It is to be noted that the terminology used herein is for the purpose of describing specific embodiments only, and is not intended to limit the exemplary embodiments according to the present disclosure. As used herein, the singular form is also intended to include the plural form unless the context clearly dictates otherwise. Furthermore, it is also to be understood that when the terms "comprising/comprise" and/or "including/include" are used in this specification, they indicate the presence of the described features, integers, steps, operations, elements, and/or components, but do not preclude the presence or addition of one or more other features, integers, steps, operations, elements, components, and/or combinations thereof.

Ordinal words such as "first" and "second" cited in this application are merely identifiers and do not have any other meaning, such as a specific order, etc. Also, for example, the term "first component" does not imply the presence of a "second component", and the term "second component" does not imply the presence of a "first component".

It is to be noted that the terms "upper," "lower," "front," "back," "left," "right," "inner," "outer," and similar expressions used herein are for illustrative purposes only and are not for limiting.

Exemplary embodiments according to the present disclosure will now be described in more detail with reference to the accompanying drawings. However, these exemplary embodiments may be implemented in many different forms and should not be construed as limited only to the embodiments set forth herein. It shall be understood that these embodiments are provided in order to make the disclosure of the present disclosure thorough and complete, and to fully convey the concept of these exemplary embodiments to those of ordinary skill in the art.

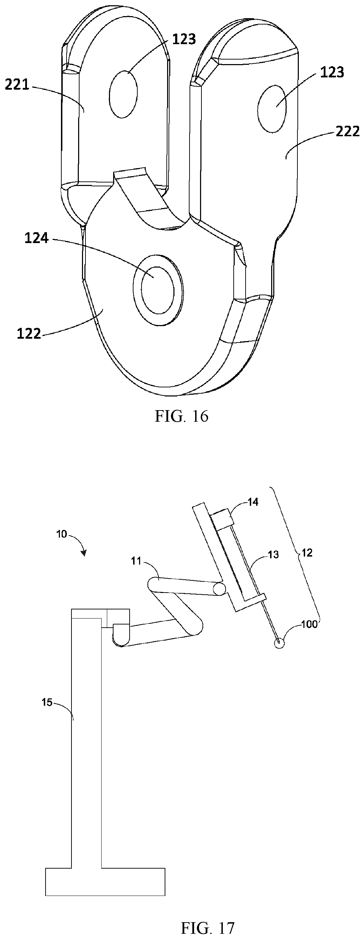

FIG. 17 is a schematic structural diagram of a surgical robot system according to an embodiment of the present disclosure. The surgical robot system includes a surgical robot 10 and a surgical instrument 12 provided in the surgical robot 10. The surgical robot 10 includes a base 15 and at least one robotic arm 11 rotatably disposed at an upper end of the base 15. Each robotic arm 11 is provided with a surgical instrument 12 at an end of the robotic arm 11. The surgical instrument 12 is detachable, to facilitate individual replacement and sterilization of the surgical instrument 12. The surgical instrument 12 includes a rear-end mechanism 14, a main shaft 13, and an end effector 100. The main shaft 13 extends from the rear-end mechanism 14 to a front end. The end effector 100 is provided at the front end of the main shaft 13. The end effector 100 is connected to the main shaft 13 via a wrist, so that the end effector 100 has at least one degree of freedom of movement relative to the main shaft 13. The rear-end mechanism 14 is configured to provide driving for the end effector 100 by a plurality of wires running through the main shaft 13. Herein, a proximal end (also referred to as a rear end) is defined as an end of the instrument away from the patient, and a distal end (also referred to as the front end) is defined as an end of the instrument closer to the patient.

To achieve a relatively small size of the wrist, in some cases, the wrist uses grooves or channels or the like to guide driving wires. However, the inventors have found that this method leads to great frictions between the wires and the grooves or channels, which in turn reduces the accuracy of motion transmission from the proximal end to the distal end, causes hysteresis in the master-slave teleoperation, and affects the surgical result. Furthermore, these frictions can also accelerate wear of the driving wires and reduce the service life of the surgical instrument.

In order to overcome or improve at least one of the above-described problems, embodiments of the present disclosure provide an end effector and a surgical instrument having the end effector.

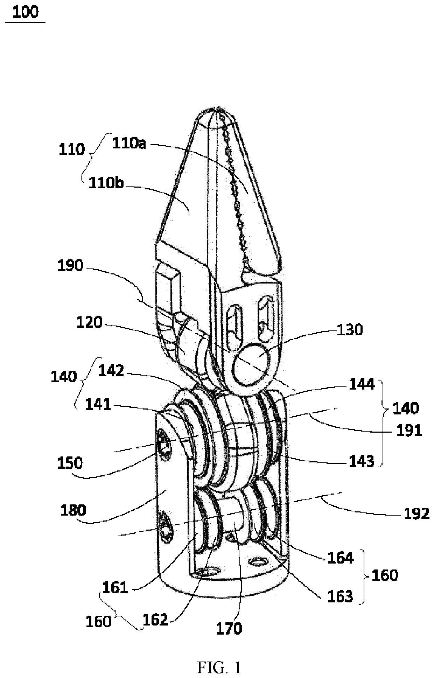

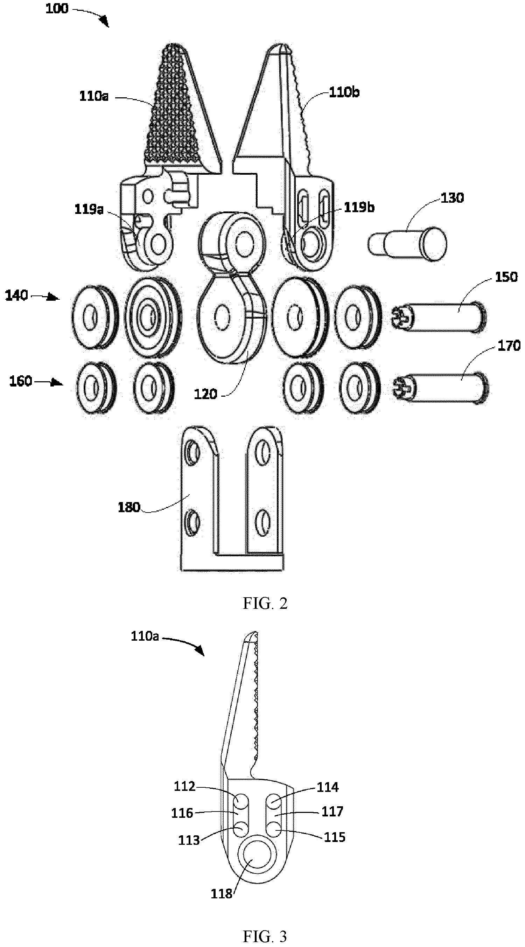

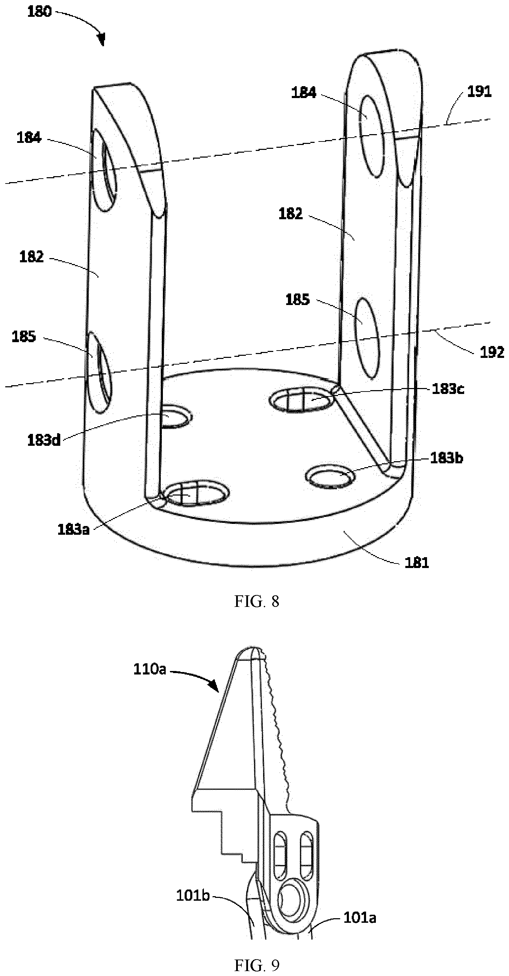

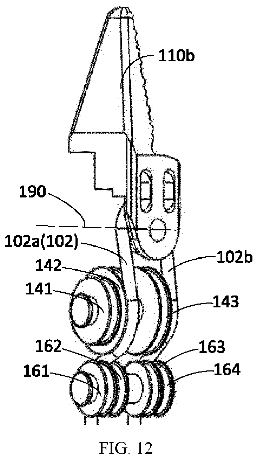

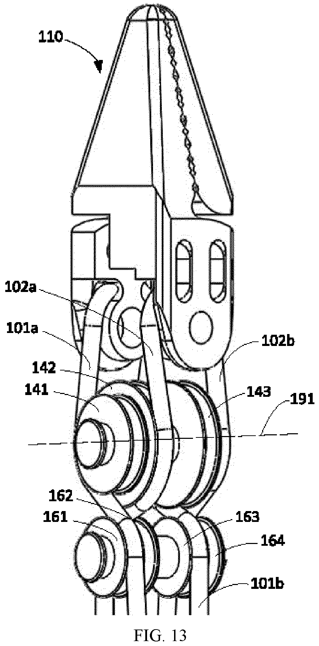

FIGS. 1 to 13 show an end effector 100 according to an embodiment of the present disclosure. The end effector 100 is applied to a surgical instrument, in particular a surgical micro-instrument. As shown in FIGS. 1 and 2, the end effector 100 may include an end effector tool 110, a middle support member 120, at least one set of guide wheels, a lower support member 180, and driving wires (not shown in FIGS. 1 and 2, referring to FIGS. 9 to 13). The end effector tool 110 may be any form of an effector, such as a pair of forceps, a pair of scissors, a clip, or the like. The end effector tool 110 is connected to the lower support member 180 through the middle support member 120. The at least one set of guide wheels is disposed on the lower support member 180, and each set of guide wheels is rotatable about a corresponding guide wheel axis of at least one guide wheel axis relative to the lower support member 180. The end effector tool 110 is rotatable about an effector axis 190. The middle support member 120 and one set of guide wheels of the at least one set of guide wheels are rotatable about one guide wheel axis of the at least one guide wheel axis. The driving wires can be wound around the end effector tool 110 and respective guide wheels of the at least one set of guide wheels.

As shown in FIGS. 1 and 2, the end effector 100 includes a pair of end effector portions at a distal end, the middle support member 120, a fork-shaped lower support member 180, ten guide wheels, and three axis pins. The pair of end effector portions are fixed to both sides of the middle support member 120 by an axis pin. The pair of end effectors is independently freely rotatable about an axis of the axis pin, to achieve a grip action, i.e., the pair of end effector portions rotates towards each other; to achieve a release action, i.e., the pair of end effector portions rotates in opposite directions; and to achieve a yaw action, i.e., the pair of end effector portions rotate in a same direction. The middle support member 120 is passed through by another axis pin and secured at a middle of the fork-shaped lower support member 180. The middle support member 120 is rotatable about an axis of the other axis pin relative to the fork-shaped lower support member 180 to achieve a pitch motion.

Hereinafter, specific structures and arrangements of the above-described components/members of the end effector 100 will be described in detail with reference to the accompanying drawings.

It is to be noted that directional terms such as "upper", "lower", "above", "below", "upward", "downward", and the like used herein to describe various components, parts, and the like of the end effector 100 are relative to the end effector 100 in a vertically placed and upright state (the placed state of the end effector 100 shown in FIG. 1).

The end effector tool 110 includes two end effector portions disposed opposite each other, including a first end effector portion 110a and a second end effector portion 110b. The two end effector portions may be provided in a shape and structure like a pair of forceps, a pair of scissors, a clip, or the like as necessary. Furthermore, the first end effector portion 110a and the second end effector portion 110b may have a substantially same structure, and are arranged in a mirror image. The driving wires include a first driving wire 101 and a second driving wire 102. The first driving wire 101 is used for the first end effector portion 110a. The second driving wire 102 is used for the second end effector portion 110b.

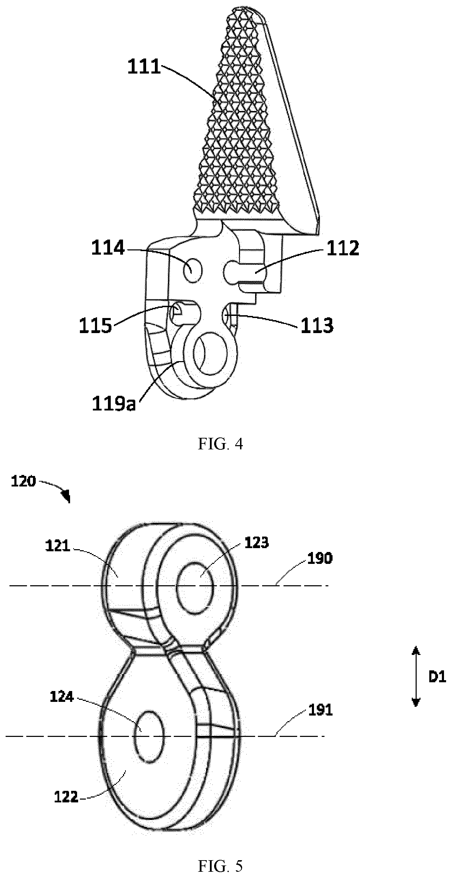

As shown in FIGS. 3 and 4, the following describes the first end effector portion 110a as an example of the end effector portion. FIG. 3 shows an outer structure of the first end effector portion 110a, and FIG. 4 shows an inner structure of the first end effector portion 110a. The first end effector portion 110a may be provided with an anti-slip structure 111 on an inner side of a head of the first end effector portion 110a, such as grooves arranged in a staggered manner, which may prevent objects, such as human tissue and surgical needles, held between the two end effector portions from sliding. The first end effector portion 110a may define, at a bottom of the first end effector portion 110a, a first threading hole 112 and a second threading hole 113 that are separated in a vertical direction, and a third threading hole 114 and a fourth threading hole 115 that are separated in the vertical direction. Each of the four threading holes is a through hole, for example, a through hole extending between an inner surface and an outer surface of the end effector tool 110.

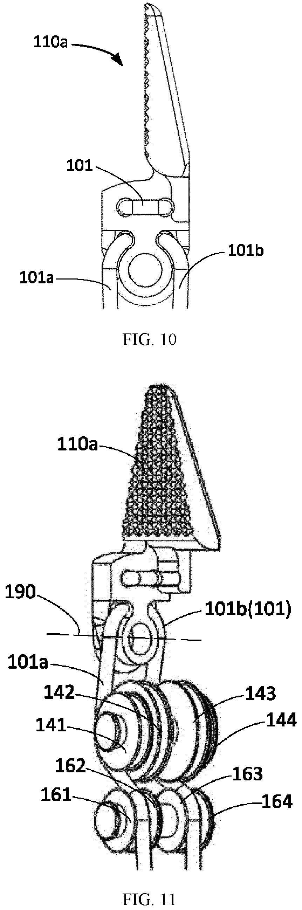

As shown in FIGS. 9 and 10, the first driving wire 101 has a first wire portion 101a and a second wire portion 101b. The first wire portion 101a passes through the first threading hole 112 from an inner side of the first end effector portion 110a and extends downward to pass through the second threading hole 113. The second wire portion 101b passes through the third threading hole 114 from the inner side of the first end effector portion 110a and extends downward to pass through the fourth threading hole 115.

As an example, in the illustrated embodiment, the first wire portion 101a and the second wire portion 101b are integrally formed. That is, the first driving wire 101 is embodied as one wire. The driving wire is repeatedly bent, for example, eight times, at an inlet and outlet of respective threading holes, resulting in plastic deformation, such that the driving wire is fixed to the end effector tool 110. In another example, mounting the driving wire on the end effector tool 110 may be achieved by using a short tube to fix the driving wire and by clamping both ends of the short tube to the end effector tool 110 to restrict movement of the driving wire relative to the end effector tool 110.

In another example, the first wire portion 101a and the second wire portion 101b are separate members. That is, the first driving wire 101 is embodied as two wires. In this embodiment, each wire is fixed to the end effector tool 110 at an end of the wire by using a fixedly connected joint (in ways such as clamping, gluing, welding, riveting, etc.) to restrict the movement of the driving wire relative to the end effector tool 110. In some embodiments, the two wires may be welded to the first end effector portion 110a at respective ends of the two wires. Alternatively, auxiliary members such as short tubes are used to clamp ends of the two wires to be snapped into corresponding threading holes or fixed to the first end effector portion 110a.

The first threading hole 112 and the third threading hole 114 correspond to each other in a horizontal direction, and the second threading hole 113 and the fourth threading hole 115 correspond to each other in the horizontal direction. Therefore, when the driving wires drive the end effector portions, the end effector portions can be balanced. The first end effector portion 110a defines a first groove 116 communicating the first threading hole 112 and the second threading hole 113, and defines a second groove 117 communicating the third threading hole 114 and the fourth threading hole 115. Both the first groove 116 and the second groove 117 are defined on an outer surface of the end effector tool 110. The driving wire is received in the first groove 116 and the second groove 117 to restrict the position of the driving wire, such that the driving wire is more firmly and reliably fixed to the end effector portion.

The manner in which the second driving wire 102 is provided in the second end effector portion 110b is substantially the same as that in which the first driving wire 101 is provided in the first end effector portion 110a, which will not be described herein for the sake of brevity. By respectively pulling and releasing two wire portions of each of the first driving wire 101 and the second driving wire 102, the first end effector portion 110a and the second end effector portion 110b can be driven to rotate about the axis of the axis pin. Furthermore, grip and yaw actions of the wrist can be realized by independently rotating the first end effector portion 110a and the second end effector portion 110b.

Compared with the conventional mounting method of the driving wires, the mounting method of the driving wires on the end effector tool 110 of the present embodiment greatly facilitates the assembly and improves the reliability of the use of the surgical instrument, which is particularly important in surgical micro-instruments.

The first end effector portion 110a may further define an effector shaft hole 118 and may be further provided with a first guide wheel portion 119a at the bottom of the first end effector portion 110a. The effector shaft hole 118 is a through hole, for example, a through hole extending between the inner surface and the outer surface of the end effector tool 110. The first guide wheel portion 119a is located on an inner side of the first end effector portion 110a. The wire portions of the first driving wire threaded inward from the second threading hole 113 and the fourth threading hole 115 can be wound to both sides of the first guide wheel portion 119a, respectively.

FIG. 5 shows the middle support member 120 of the present embodiment. As shown in FIG. 5, the middle support member 120 extends from the proximal end to the distal end. In other words, the middle support member 120 extends in an axis direction of the instrument. The axis direction of the instrument includes a direction extending from the proximal end to the distal end of the instrument and a direction extending from the distal end to the proximal end of the instrument, for example, in a first direction D1 of FIG. 5. The end effector tool 110, the middle support member 120, the at least one set of guide wheels, and the lower support member 180 are arranged in a longitudinal direction of the end effector 100. The longitudinal direction of the end effector 100 is parallel to a vertical direction in a placed state of the end effector 100 shown in FIG. 1. The "first direction D1" herein refers to a direction substantially the same as a proximal-distal direction of the surgical instrument or the longitudinal direction of the end effector 100, or refers to a direction parallel to the vertical direction in the placed state of the end effector 100 shown in FIG. 1.

The middle support member 120 includes a first support portion 121 for defining an effector axis 190 and a second support portion 122 for defining a guide wheel axis. The end effector tool 110 and the first support portion 121 are rotatable about the effector axis 190. The second support portion 122 and one set of guide wheels of the at least one set of guide wheels are rotatable about the guide wheel axis. In some embodiments, the first support portion 121 defines a first middle support hole 123. The first middle support hole 123 has a centerline for defining the effector axis 190. In other words, the centerline of the first middle support hole 123 is collinear with the effector axis 190. The second support portion 122 defines a second middle support hole 124. The second middle support hole 124 has a centerline for defining the guide wheel axis. In other words, the centerline of the first middle support hole 123 is collinear with the guide wheel axis.

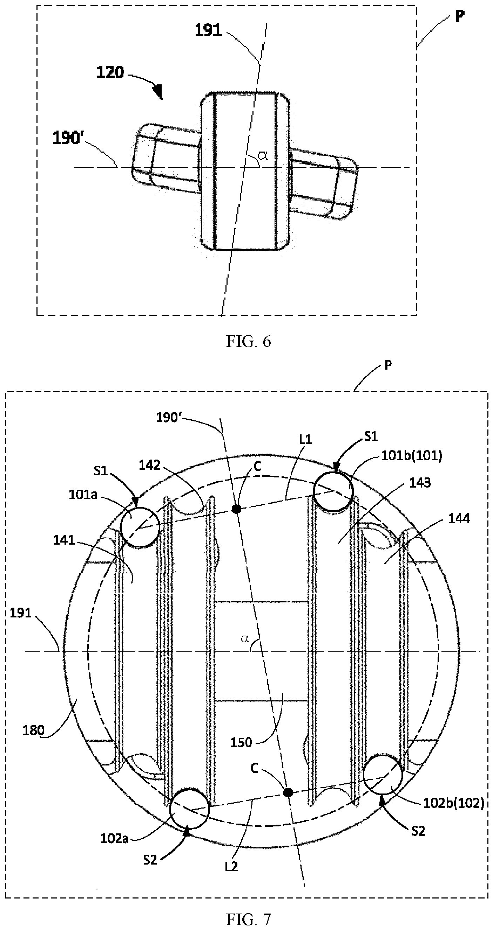

The effector axis 190 and the guide wheel axis defined by the second support portion 122 are skew lines, and an included angle α between the effector axis 190 and the guide wheel axis is an acute angle. Further from another aspect, as shown in FIGS. 6 and 7, the effector axis 190 has a projection 190' (effector axis projection 190') on a horizontal cross-section P of the set of guide wheels rotatable about the guide wheel axis defined by the second support portion 122. The guide wheel axis defined by the second support portion 122 is on the horizontal cross-section P. The projection 190' of the effector axis is non-orthogonal to the guide wheel axis 191 defined by the second support portion 122, or in other words, the included angle α between the projection 190' of the effector axis and the guide wheel axis 191 is an acute angle. At a pitch angle of 0°, both the effector axis 190 and the guide wheel axis 191 defined by the second support portion 122 are perpendicular to an axis direction of the instrument. The axis direction of the instrument refers to the proximal-distal direction of the instrument. The pitch angle refers to an angle at which the end effector tool 110 rotates about the guide wheel axis 191 or a pitch axis 191 and deviates from the axis of the instrument, and a yaw angle refers to an angle at which the end effector tool 110 rotates about the effector axis 190 or a yaw axis 190 and deviates from the axis of the instrument.

It will be appreciated that the effector axis 190 herein corresponds to the yaw axis and the guide wheel axis defined by the second support portion 122 corresponds to the pitch axis.

By arranging both the pitch axis and the yaw axis as skew lines that are not perpendicular to each other, it is possible to ensure that the driving wires extending downward from the end effector tool 110 to the at least one set of guide wheels are tangent to guiding structures of respective guide wheels with which respective driving wires cooperate. Therefore, when the end effector tool 110 is operated, the driving wires and the guiding structures of the respective guide wheels are subjected to less force at respective initial contact positions, thereby generating less friction and reducing the wear of the driving wires.

The first support portion 121 and the second support portion 122 are integrally formed. Alternatively, the first support portion 121 and the second support portion 122 are separate members. As shown in FIGS. 5 and 6, both the first support portion 121 and the second support portion 122 are configured in a cylindrical shape. The first support portion 121 and the second support portion 122 are connected at respective cylindrical surfaces of the first support portion 121 and the second support portion 122. The first support portion 121 is located between the first end effector portion 110a and the second end effector portion 110b. The middle support member 120 is configured in a shape similar to the figure "8" in torsion.

The first middle support hole 123 is spaced apart from the second middle support hole 124 in the first direction D1, such that the effector axis 190 is spaced apart from the guide wheel axis defined by the second support portion 122 in the first direction D1.

In the illustrated embodiments, the at least one set of guide wheels includes an upper guide wheel set 140 and a lower guide wheel set 160. The upper guide wheel set 140 is rotatable about a first guide wheel axis 191 relative to the lower support member 180. The lower guide wheel set 160 is rotatable about a second guide wheel axis 192 relative to the lower support member 180. The middle support member 120 is disposed coaxially with the upper guide wheel set 140, i.e., both the middle support member 120 and the upper guide wheel set 140 are rotatable about the first guide wheel axis 191. The first guide wheel axis 191 may be parallel to the second guide wheel axis 192. In this embodiment, the second support portion 122 defines the first guide wheel axis 191, and the second support portion 122 and the upper guide wheel set 140 are rotatable about the first guide wheel axis 191. The centerline of the second middle support hole 124 defines the first guide wheel axis 191. In other words, the centerline of the first middle support hole 123 is collinear with the first guide wheel axis 191.

The upper guide wheel set 140 includes a first upper guide wheel 141 and a second upper guide wheel 142 located on a side of two opposite sides of the second support portion 122, and a third upper guide wheel 143 and a fourth upper guide wheel 144 located on another side of the two opposite sides of the second support portion 122. The first upper guide wheel 141 is located outside the second upper guide wheel 142, and the fourth upper guide wheel 144 is located outside the third upper guide wheel 143. That is, the first upper guide wheel 141 and the fourth upper guide wheel 144 are regarded as outer guide wheels, and the second upper guide wheel 142 and the third upper guide wheel 143 are regarded as inner guide wheels. Furthermore, the second support portion 122 is located between the second upper guide wheel 142 and the third upper guide wheel 143. The second upper guide wheel 142 has a diameter that may be larger than a diameter of the first upper guide wheel 141, and the third upper guide wheel 143 has a diameter that may be larger than a diameter of the fourth upper guide wheel 144. That is, the upper guide wheels closer to an inner side of the instrument may be larger than the upper guide wheels closer to an outer side of the instrument. The diameter of the first upper guide wheel 141 may be equal to the diameter of the fourth upper guide wheel 144, and the diameter of the second upper guide wheel 142 may be equal to the diameter of the third upper guide wheel 143.

According to the schemes, within an allowable diameter range of the surgical instrument, by selecting the relatively large-diameter guide wheels on the inner side of the wrist and using the relatively small-diameter guide wheels on the outer side of the wrist, the diameter of each of the guide wheels of the upper guide wheel set may be increased as much as possible, thereby increasing the joint stiffness of the pitch joint and the service life of the driving wires, which is even more important in the surgical micro-instruments (e.g., the instrument with the diameter of less than 4 mm).

Further, two additional driving wires may be, respectively, fixed on both sides of the second support portion 122 perpendicular to the pitch axis 191 and along the axis direction of the instrument to enhance the stability and strength of the pitch movement of the end effector tool and increase the stiffness of the wrist.

The lower guide wheel set 160 includes a first lower guide wheel 161, a second lower guide wheel 162, a third lower guide wheel 163, and a fourth lower guide wheel 164 arranged sequentially. The first lower guide wheel 161 is located outside the second lower guide wheel 162, and the fourth lower guide wheel 164 is located outside the third lower guide wheel 163. That is, the first lower guide wheel 161 and the fourth lower guide wheel 164 are regarded as outer guide wheels, and the second lower guide wheel 162 and the third lower guide wheel 163 are regarded as inner guide wheels. The second lower guide wheel 162 and the third lower guide wheel 163 may be spaced apart from each other by a shaft sleeve. The first lower guide wheel 161 has a diameter equal to or larger than a diameter of the second lower guide wheel 162, and the fourth lower guide wheel 164 has a diameter equal to or larger than a diameter of the third lower guide wheel 163. That is, the upper guide wheels closer to the outer side of the instrument may be equal to or larger than the upper guide wheels closer to the inner side of the instrument. The diameter of the first lower guide wheel 161 may be equal to the diameter of the fourth lower guide wheel 164, and the diameter of the second lower guide wheel 162 may be equal to the diameter of the third lower guide wheel 163.

Each guide wheel in the guide wheel set has a guiding structure for the corresponding driving wire. The guiding structure may include any suitable guiding structure such as a groove or a channel. With aid of the guiding structure, each wire portion of the driving wire can be guided separately to reduce wear of the driving wire during guiding.

According to the present embodiment, a novel four-wire-driving wrist mechanism with guide wheels is provided, which includes 8 guide wheels and 2 guide wheel portions, and the two wire portions of the first driving wire 101 and the two wire portions of the second driving wire 102 are enveloped by a maximum diameter size allowed by the wrist along a radial direction of the wrist after being guided by corresponding guide wheels of the upper guide wheel set 140, so as to maximize the overall diameter of the upper guide wheel set 140, which enables the four-wire-driving wrist mechanism with the guide wheels to achieve higher stiffness at a small dimension allowed (e.g., allowed diameter being less than 4 mm).

As shown in FIG. 7, the first guide wheel axis 191 is located on the horizontal cross-section P of the upper guide wheel set 140. Furthermore, the horizontal cross-section P extends through the centerline of the second middle support hole 124 and a centerline of each guide wheel of the upper guide wheel set 140. A cross section of the first driving wire 101 along the horizontal cross-section P is regarded as a first driving wire cross section S1, and a cross section of the second driving wire 102 along the horizontal cross-section P is regarded as a second driving wire cross section S2. A line connecting both a center of the first driving wire cross section S1 corresponding to the first upper guide wheel 141 and a center of the first driving wire cross section S1 corresponding to the third upper guide wheel 143 constitutes a first reference line L1. Furthermore, a line connecting both a center of a cross section of the first wire portion 101a of the first driving wire 101 and a center of a cross section of the second wire portion 101b of the first driving wire 101 constitutes the first reference line L1. A line connecting both a center of the second driving wire cross section S2 corresponding to the second upper guide wheel 142 and a center of the second driving wire cross section S2 corresponding to the fourth upper guide wheel 144 constitutes a second reference line L2. Furthermore, a line connecting both a center of a cross section of a first wire portion 102a of the second driving wire 102 and a center of a cross section of the second wire portion 102b of the second driving wire 102 constitutes the second reference line L2.

The first reference line L1 is parallel to the second reference line L2, and midpoints C of both the first reference line L1 and the second reference line L2 are used to position the effector axis. For example, both the midpoint of the first reference line L1 and the midpoint of the second reference line L2 lie on the projection 190' of the effector axis. That is, the line connecting the midpoint of the first reference line L1 and the midpoint of the second reference line L2 coincides with the projection 190' of the effector axis. In practical application, the line connecting the midpoints can also have a slight deviation from the projection 190' of the effector axis, or there may be a suitable deviation angle between the line connecting the midpoints and the projection 190' of the effector axis according to the practical application scenarios. In other words, in these cases, the line connecting the midpoint of the first reference line L1 and the midpoint of the second reference line L2 deviates from the projection 190' of the effector axis. Thus, a position of the effector axis 190 may be defined by two points, namely, the midpoint C of the line connecting the center of the cross section of the first wire portion 101a and the center of the cross section of the second wire portion 101b of the first driving wire 101, and the midpoint C of the line connecting the center of the cross section of the first wire portion 102a and the center of the cross section of the second wire portion 102b of the second driving wire 102. A position of the first guide wheel portion 119a can be determined according to positions of the first wire portion 101a and the second wire portion 101b of the first driving wire 101, such that a projection of the first guide wheel portion 119a on the horizontal cross-section P is located on the first reference line L1. Therefore, the first driving wire 101 is secured to be tangent to the guiding structure such as the channel of the first guide wheel portion 119a, the first upper guide wheel 141, and the third upper guide wheel 143, to reduce wear of the driving wire. Similarly, a position of a second guide wheel portion 119b may be determined according to positions of a first wire portion 102a and a second wire portion 102b of the second driving wire 102, such that a projection of the second guide wheel portion 119b on the horizontal cross-section P is located on the second reference line L2. Therefore, the second driving wire 102 is secured to be tangent to the guiding structure such as the channel of the second guide wheel portion 119b, the second upper guide wheel 142, and the fourth upper guide wheel 144 to reduce wear of the driving wire. In the actual design, in consideration of manufacturability of the components/members/parts and the like, the positions of the first guide wheel portion 119a and the second guide wheel portion 119b can be appropriately adjusted at the expense of a small amount of the service life of the driving wires. For example, relative positions of the first guide wheel portion 119a and the second guide wheel portion 119b can be made closer to each other. Furthermore, diameters of the first guide wheel portion 119a and the second guide wheel portion 119b may be appropriately increased to increase the rigidity at the degree of freedom of grip and yaw, which can also reduce the axial force exerted on the guide wheels by the pulling force of the driving wires. In an ideal state, no axial force may be generated, thereby reducing the axial friction caused by the axial force.

As shown in FIG. 8, the lower support member 180 includes a bottom wall 181 and two upwardly extending side walls 182, in such a manner that the lower support member 180 has a fork-shaped structure. The bottom wall 181 defines wire passing holes through which the driving wires pass. The wire passing holes include a first wire passing hole 183a, a second wire passing hole 183b, a third wire passing hole 183c, and a fourth wire passing hole 183d that are arranged at intervals. The first and second wire passing holes 183a, 183b are located on a side of the second guide wheel axis 192, and the third and fourth wire passing holes 183c, 183d are located on the other side of the second guide wheel axis 192. The first and fourth wire passing holes 183a, 183d are close to one of the two side walls 182, and the second and third wire passing holes 183b, 183c are close to the other of the two side walls 182. The upper guide wheel set 140 and the lower guide wheel set 160 are disposed between the two side walls 182, to define the upper guide wheel set 140 and the lower guide wheel set 160 within the fork-shaped structure. Each side wall 182 of the two side walls 182 defines a first lower support hole 184 and a second lower support hole 185 for mounting the axis pins.

The end effector 100 further includes an effector axis pin 130, a first guide wheel axis pin 150, and a second guide wheel axis pin 170. The end effector tool 110 is rotatably disposed on the effector axis pin 130. The effector axis pin 130 passes through the first middle support hole 123. Therefore, the first end effector portion 110a, the first support portion 121, and the second end effector portion 110b are connected together by the effector axis pin 130. The upper guide wheel set 140 is rotatably disposed on the first guide wheel axis pin 150. The first guide wheel axis pin 150 passes through the second middle support hole 124. The first guide wheel axis pin 150 is fixed to the first lower support holes 184 to support the upper guide wheel set 140. Therefore, the upper guide wheel set 140 and the second support portion 122 are attached to the lower support member 180 by the first guide wheel axis pin 150. The lower guide wheel set 160 is rotatably disposed on the second guide wheel axis pin 170, and the second guide wheel axis pin 170 is fixed to the second lower support holes 185 to support the lower guide wheel set 160. Therefore, the lower guide wheel set 160 is attached to the lower support member 180 by the second guide wheel axis pin 170.

According to the present aspect, the effector axis pin 130 passes through the first end effector portion 110a, the middle support member 120, and the second end effector portion 110b sequentially, to restrict the middle support member 120 between the first end effector portion 110a and the second end effector portion 110b located on both sides of the middle support member 120. The first guide wheel axis pin 150 passes through the middle support member 120, the upper guide wheel set 140, and the lower support member 180 concurrently, to restrict the first and second upper guide wheels to one side of the middle support member 120 and restrict the third and fourth upper guide wheels to the other side of the middle support member 120, and to restrict the upper guide wheel set 140 between the two side walls 182 of the lower support member 180. The second guide wheel axis pin 170 passes through the lower guide wheel set 160 and the lower support member 180 concurrently, to restrict the lower guide wheel set 160 between the two side walls182 of the lower support member 180.

As shown in FIG. 2, the effector axis pin 130 may be configured as a straight stepped shaft, whereby an axial clearance fit between the two end effector portions, the middle support member, and the effector axis pin 130 can be guaranteed without the need for additional mounting fixtures/jigs by appropriate selection of tolerances, thereby greatly improving machining processability for maintenance and assembly. In some embodiments, the effector axis pin 130 is configured in a stepped shape and includes a large end, a middle portion, and a small end that sequentially decrease in diameter. The first end effector portion 110a is sleeved on and fixed to the small end, which may be achieved by methods including, but not limited to, gluing, welding, and interference fit. Both of the second end effector portion 110b and the middle support member 120 are sleeved on the middle portion in a clearance fit. For example, the middle portion is in a clearance fit with the hole of the second end effector portion 110b and the hole of the middle support member 120. Both of the second end effector portion 110b and the middle support member 120 are rotatable relative to the middle portion. The large end is located on outside the second end effector portion 110b and abuts the second end effector portion 110b, which is used to restrict the second end effector portion 110b from moving outward. By controlling the length and the dimension/size of the middle portion of the effector axis pin 130, the axial clearance between the first end effector portion 110a, the middle support member 120, the second end effector portion 110b, and the effector axis pin 130 can be guaranteed without additional mounting jigs when connecting the effector axis pin 130 and the first end effector portion 110a. The effector axis pin 130 of this solution passes through the middle support member 120, the upper guide wheel set 140, and the lower support member 180 concurrently, to restrict the first and second upper guide wheels to one side of the middle support member 120 and restrict the third and fourth upper guide wheels to the other side of the middle support member 120, and to restrict the upper guide wheel set 140 between the two side walls 182 of the lower support member 180.

The guide wheel axis pin can be configured as a straight stepped shaft, and thus, with appropriate tolerance selection, a clearance fit between the respective guide wheels of each guide wheel set, the lower support member 180, and the guide wheel axis pin can be guaranteed without the need for additional mounting fixtures, thereby greatly improving machining workability for maintenance and assembly. In some embodiments, both the guide wheel axis pins described above are configured in a stepped shape and each include a large end, a middle portion, and a small end that sequentially decrease in diameter. On a same guide wheel axis pin, a pair of inner guide wheels and a pair of outer guide wheels are sleeved on the middle portion of the axis pin, and the small end of the guide wheel axis pin is fixed in the hole of the lower support member 180, which may be achieved by methods including, but not limited to, gluing, welding, and interference fit. By controlling the length and the dimension/size of the middle portion of the guide wheel axis pin, the axial clearance between the respective guide wheels of each guide wheel set, the lower support member 180, and the guide wheel axis pin can be guaranteed without the need for additional mounting fixtures when connecting the guide wheel axis pin and the lower support member 180.

As shown in FIGS. 11, 12, and 13, the first driving wire 101 rides on the first guide wheel portion 119a of the first end effector portion 110a. The first wire portion 101a of the first driving wire 101 extends downward from one side of the first guide wheel portion 119a and rides on the first upper guide wheel 141. The second wire portion 101b of the first driving wire 101 extends downward from the other side of the first guide wheel portion 119a and rides on the third upper guide wheel 143. The second driving wire 102 rides on the second guide wheel portion 119b of the second end effector portion 110b. The first wire portion 102a of the second driving wire 102 extends downward from one side of the second guide wheel portion 119b and rides on the second upper guide wheel 142. The second wire portion 102b of the second driving wire 102 extends downward from the other side of the second guide wheel portion 119b and rides on the fourth upper guide wheel 144.

The first wire portion 101a of the first driving wire 101 extending downward from the first upper guide wheel 141 passes between the first upper guide wheel 141 and the first lower guide wheel 161 and extends downward to ride on the first lower guide wheel 161. The second wire portion 101b of the first driving wire 101 extending downward from the third upper guide wheel 143 passes between the third upper guide wheel 143 and the third lower guide wheel 163 and extends downward to ride on the third lower guide wheel 163. The first wire portion 102a of the second driving wire 102 extending downward from the second upper guide wheel 142 passes between the second upper guide wheel 142 and the second lower guide wheel 162 and extends downward to ride on the second lower guide wheel 162. The second wire portion 102b of the second driving wire 102 extending downward from the fourth upper guide wheel 144 passes between the fourth upper guide wheel 144 and the fourth lower guide wheel 164 and extends downward to ride on the fourth lower guide wheel 164.

The first wire portion 101a of the first driving wire 101 extending downward from the first lower guide wheel 161 passes downward through (extends downward to pass through) the first wire passing hole 183a. The second wire portion 101b of the first driving wire 101 extending downward from the third lower guide wheel 163 passes downward through the second wire passing hole 183b. The first wire portion 102a of the second driving wire 102 extending downward from the second lower guide wheel 162 passes downward through the fourth wire passing hole 183d. The second wire portion 102b of the second driving wire 102 extending downward from the fourth lower guide wheel 164 passes downward through the third wire passing hole 183c. Therefore, the driving wires extends into the interior of the instrument shaft after passing through the wire passing holes of the lower support member 180.

According to this solution, the first upper guide wheel 141, the second upper guide wheel 142, the third upper guide wheel 143, and the fourth upper guide wheel 144 are parallel to the first lower guide wheel 161, the second lower guide wheel 162, the third lower guide wheel 163, and the fourth lower guide wheel 164, respectively, to guide the first wire portion 101a of the first driving wire 101, the first wire portion 102a of the second driving wire 102, the second wire portion 101b of the first driving wire 101, and the second wire portion 102b of the second driving wire 102, respectively, so as to reduce wear in the driving wires in the guiding process. In addition, the first wire portion 101a and the second wire portion 101b of the first driving wire 101 are pulled in and the first wire portion 102a and the second wire portion 102b of the second driving wire 102 are released concurrently, such that forward rotation about the first guide wheel axis 191 can be realized. On the contrary, the first wire portion 101a and the second wire portion 101b of the first driving wire 101 are released and the first wire portion 102a and the second wire portion 102b of the second driving wire 102 are pulled in concurrently, reverse rotation can be realized. The first wire portion 102a and the second wire portion 102b of the second driving wire 102, and the second wire portion 101b and the first wire portion 101a of the first driving wire 101 are guided by the upper guide wheel set 140 and then guided by the lower guide wheel set 160, and finally extends into the interior of the instrument shaft after passing through the fourth wire passing hole 183d, the third wire passing hole 183c, the second wire passing hole 183b, and the first wire passing hole 183a, respectively. The arrangement of the lower guide wheel set 160 is to ensure that within a range (for example, within the range of -90° to 90°) of a motion angle of the pitch join, the first wire portion 101a of the first driving wire 101 always rides on the first upper guide wheel 141, the second wire portion 101b of the first driving wire 101 always ride on the third upper guide wheel 143, the first wire portion 102a of the second driving wire 102 always rides on the second upper guide wheel 142, and the second wire portion 102b of the second driving wire 102 always rides on the fourth upper guide wheel 144. In this way, it is possible to ensure that there is a linear relationship between the motion angle of the pitch joint and the change in the length of the driving wires, which facilitates the design of the rear-end driving mechanism.

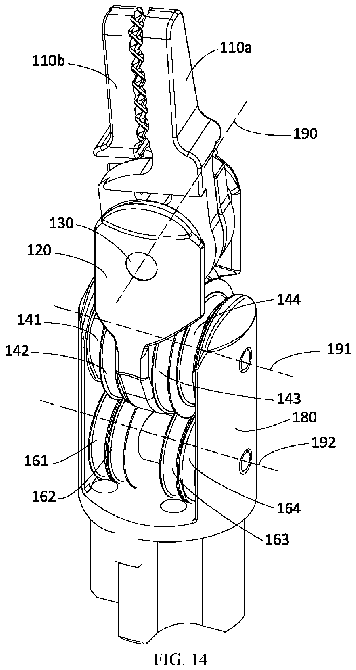

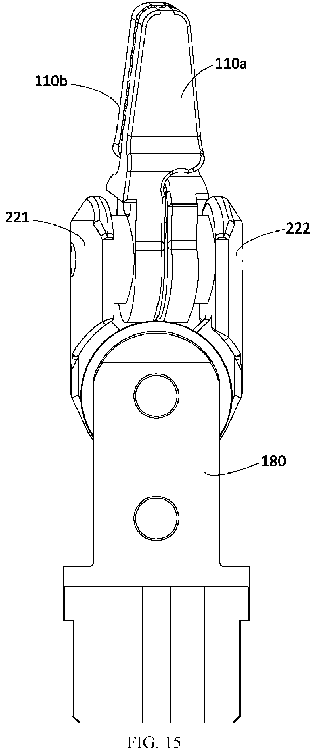

In other embodiments, the middle support member 120 may also have a configuration different from that shown in FIGS. 5 and 6. For example, in embodiments shown in FIGS. 14 to 16, the middle support member 120 may be configured as a fork-shaped structure. In this embodiment, the first support portion 121 may include a first arm portion 221 and a second arm portion 222. The first arm portion 221 and the second arm portion 222 are spaced apart from each other and extend in a same direction. Both the first arm portion 221 and the second arm portion 222 define a first middle support hole 123. The second support portion 122 is configured in a cylindrical shape and defines a second middle support hole 124. The first end effector portion 110a and the second end effector portion 110b can be located between the first arm portion 221 and the second arm portion 222, and are fixed to the first arm portion 221 and the second arm portion 222 by an effector axis pin. The second support portion 122 is located between the second upper guide wheel 142 and the third upper guide wheel 143, and is fixed to the lower support member 180 by a guide wheel axis pin.

In other embodiments not shown, the first support portion 121 is configured in a cylindrical shape and is disposed between the first end effector portion 110a and the second end effector portion 110b. The second support portion 122 includes a first arm portion 221 and a second arm portion 222. The first arm portion 221 and the second arm portion 222 are spaced apart from each other and extend in a same direction. The first arm portion 221 may be located between the first upper guide wheel 141 and the second upper guide wheel 142, and the second arm portion 222 may be located between the third upper guide wheel 143 and the fourth upper guide wheel 144. Alternatively, the first arm portion 221 may be located between the first upper guide wheel 141 and one side wall 182 of the lower support member 180, and the second arm portion 222 may be located between the fourth upper guide wheel 144 and the other side wall 182 of the lower support member 180. Alternatively, the first arm portion 221 and the second arm portion 222 are located outside the lower support member 180.

Furthermore, in other embodiments, the first end effector portion 110a and the second end effector portion 110b are combined into one end effector portion to reduce one degree of freedom to achieve conversion of the 3-degree-of-freedom wrist to a 2-degree-of-freedom wrist, and the end effector tool 110 may be replaced with a charged energy burning head, a laser fiber, or the like.

According to another aspect of the present disclosure, there is provided a surgical instrument, including the end effector 100 according to any of the embodiments described above.

Unless otherwise defined, technical and scientific terms used herein have the same meaning as commonly understood by those skilled in the art of the present disclosure. The terminology used herein is for the purpose of describing specific implementations only and is not intended to limit the present disclosure. Features described herein in one embodiment may be applied to another embodiment, alone or in combination with other features, unless the feature is not applicable in the other embodiment or otherwise stated.

The present disclosure has been described with reference to the above-described embodiments, but it shall be understood that the above-described embodiments are merely for the purpose of illustration and explanation, and the present disclosure is not limited to the above-described embodiments, and many more modifications and modifications can be made according to the teachings of the present disclosure, and these modifications and modifications fall within the scope of protection claimed in the present disclosure.

## Claims

1. An end effector of a surgical instrument, comprising:
a lower support member;
at least one set of guide wheels disposed on the lower support member, wherein each set of guide wheels is rotatable about a corresponding guide wheel axis of at least one guide wheel axis relative to the lower support member;
a middle support member, including a first support portion for defining an effector axis and a second support portion for defining one guide wheel axis of the at least one guide wheel axis, wherein the second support portion and one set of guide wheels of the at least one set of guide wheels are rotatable about the one guide wheel axis, wherein the first support portion is disposed in a distal direction of the second support portion; and
an end effector tool, wherein the end effector tool is connected to the lower support member through the middle support member, and the end effector tool and the first support portion are rotatable about the effector axis;
wherein the effector axis and the one guide wheel axis are skew lines, and an included angle between the effector axis and the one guide wheel axis is an acute angle.

2. The end effector of claim 1, wherein at a pitch angle of 0°, both the effector axis and the one guide wheel axis are perpendicular to an axis direction of the instrument, where the axis direction of the instrument refers to a proximal-distal direction of the instrument.

3. The end effector of claim 1 or 2, wherein the end effector tool includes a first end effector portion and a second end effector portion that are opposite to each other, and each of the first end effector portion and the second end effector portion is rotatably connected to the first support portion about the effector axis.

4. The end effector of claim 3, further comprising driving wires, wherein each of the first end effector portion and the second end effector portion defines a first threading hole and a second threading hole that are separated in a vertical direction, and a third threading hole and a fourth threading hole that are separated in the vertical direction;
wherein each respective driving wire of the driving wires has a first wire portion and a second wire portion, wherein the first wire portion passes through, from an inner side of a corresponding end effector portion of the first end effector portion and the second end effector portion, the first threading hole and extends downward to pass through the second threading hole, and wherein the second wire portion passes through, from the inner side of the corresponding end effector portion, the third threading hole and extends downward to pass through the fourth threading hole.

5. The end effector of claim 4, wherein the first threading hole corresponds to the third threading hole in a horizontal direction, and the second threading hole corresponds to the fourth threading hole in the horizontal direction.

6. The end effector of claim 4 or 5, wherein the corresponding end effector portion defines a first groove communicating the first threading hole and the second threading hole, and defines a second groove communicating the third threading hole and the fourth threading hole, wherein the first groove and the second groove are defined on an outer surface of the corresponding end effector portion, and the respective driving wire is received in the first groove and the second groove.

7. The end effector of claim 3, wherein the at least one set of guide wheels includes an upper guide wheel set,
wherein the upper guide wheel set is rotatable about a first guide wheel axis relative to the lower support member;
wherein the upper guide wheel set includes a first upper guide wheel and a second upper guide wheel that are located on a side of the second support portion, and a third upper guide wheel and a fourth upper guide wheel that are located on another side of the second support portion; and
wherein the first upper guide wheel is located outside the second upper guide wheel and the fourth upper guide wheel is located outside the third upper guide wheel.

8. The end effector of claim 7, further comprising a first driving wire and a second driving wire;
wherein the first driving wire rides on a first guide wheel portion of the first end effector portion and the first driving wire has a first wire portion and a second wire portion, wherein the first wire portion of the first driving wire extends downward from a side of the first guide wheel portion and rides on the first upper guide wheel, and the second wire portion of the first driving wire extends downward from another side of the first guide wheel portion and rides on the third upper guide wheel; and
wherein the second driving wire rides on a second guide wheel portion of the second end effector portion, and the second driving wire has a first wire portion and a second wire portion, wherein the first wire portion of the second driving wire extends downward from a side of the second guide wheel portion and rides on the second upper guide wheel, and the second wire portion of the second driving wire extends downward from another side of the second guide wheel portion and rides on the fourth upper guide wheel.

9. The end effector of claim 7 or 8, wherein the second upper guide wheel has a diameter larger than a diameter of the first upper guide wheel, and the third upper guide wheel has a diameter larger than a diameter of the fourth upper guide wheel.

10. The end effector of claim 8 or 9, wherein:
the first guide wheel axis is on a horizontal cross-section of the upper guide wheel set, the first driving wire has a first driving wire cross section along the horizontal cross-section, and the second driving wire has a second driving wire cross section along the horizontal cross-section;
a line connecting a center of the first driving wire cross section corresponding to the first upper guide wheel and a center of the first driving wire cross section corresponding to the third upper guide wheel constitutes a first reference line;
a line connecting both a center of the second driving wire cross section corresponding to the second upper guide wheel and a center of the second driving wire cross section corresponding to the fourth upper guide wheel constitutes a second reference line; and
the first reference line is parallel to the second reference line, and a line connecting a midpoint of the first reference line and a midpoint of the second reference line coincides with or deviates from a projection of the effector axis on the horizontal cross-section.

11. The end effector of claim 10, wherein a projection of a first guide wheel portion on the horizontal cross-section is located on the first reference line and a projection of the second guide wheel portion on the horizontal cross-section is located on the second reference line.

12. The end effector of any one of claims 8 to 11, wherein the at least one set of guide wheels further comprises a lower guide wheel set, and the lower guide wheel set is rotatable about a second guide wheel axis relative to the lower support member, wherein
the lower guide wheel set includes a first lower guide wheel, a second lower guide wheel, a third lower guide wheel, and a fourth lower guide wheel; and
the first lower guide wheel is located outside the second lower guide wheel, and the fourth lower guide wheel is located outside the third lower guide wheel.

13. The end effector of claim 12, wherein the first driving wire extending downward from the first upper guide wheel passes between the first upper guide wheel and the first lower guide wheel and extends downward to ride on the first lower guide wheel, and the first driving wire extending downward from the third upper guide wheel passes between the third upper guide wheel and the third lower guide wheel and extends downward to ride on the third lower guide wheel;
wherein the second driving wire extending downward from the second upper guide wheel passes between the second upper guide wheel and the second lower guide wheel and extends downward to ride on the second lower guide wheel, and the second driving wire extending downward from the fourth upper guide wheel passes between the fourth upper guide wheel and the fourth lower guide wheel and extends downward to ride on the fourth lower guide wheel.

14. The end effector of claim 12 or 13, wherein the first lower guide wheel has a diameter equal to or greater than a diameter of the second lower guide wheel, and the fourth lower guide wheel has a diameter equal to or greater than a diameter of the third lower guide wheel.

15. The end effector of any one of claims 12 to 14, wherein the lower support member includes a bottom wall and two upwardly extending side walls, wherein the bottom wall defines wire passing holes through which the driving wires pass, wherein
the upper guide wheel set and the lower guide wheel set are disposed between the two side walls; and
each of the two side walls defines a first lower support hole and a second lower support hole, the upper guide wheel set is supported by a guide wheel axis pin fixed to the first lower support holes, and the lower guide wheel set is supported by another guide wheel axis pin fixed to the second lower support holes.

16. The end effector of any one of claims 3 to 15, wherein the first support portion and the second support portion are each configured in a cylindrical shape, the first support portion and the second support portion are connected at respective cylindrical surfaces of the first support portion and the second support portion, and the first support portion is disposed between the first end effector portion and the second end effector portion.

17. The end effector of claim 16, further comprising an effector axis pin, wherein the first support portion defines a first middle support hole, the effector axis pin passes through the first middle support hole, and the effector axis pin is configured in a stepped shape and includes a large end, a middle portion, and a small end that are sequentially decreased in diameter;
wherein the first end effector portion is sleeved on and fixed to the small end, wherein the first end effector portion and the middle support member are sleeved on the middle portion in a clearance fit and rotatable relative to the middle portion, and wherein the large end is located outside the second end effector portion and abuts the second end effector portion.

18. The end effector of any one of claims 1 to 15, wherein the first support portion includes a first arm portion and a second arm portion spaced apart from the first arm portion along the effector axis, and the end effector tool is disposed between the first arm portion and the second arm portion.

19. The end effector of any one of claims 1 to 18, wherein at least one pitch driving wire is fixed on a corresponding side of both sides of the second support portion perpendicular to the one guide wheel axis and along an axis direction of the instrument.

20. A surgical instrument, comprising the end effector of any one of claims 1 to 19.
